# EUROPÄISCHE PATENTANMELDUNG

(11) **EP 3 915 701 A1**
(43) Veröffentlichungstag der Anmeldung: **01.12.2021**
(21) Anmeldenummer: 20177036.9
(22) Anmeldetag: 28.05.2020
(51) Int. Cl.: B22F 3/105, B22F 3/22, G06F 30/23, G16C 60/00, B33Y 50/00

(54) **SIMULATIONSSYSTEM ZUR AUSWAHL EINER LEGIERUNG SOWIE EINES FERTIGUNGSVERFAHRENS FÜR EIN ZU FERTIGENDES WERKSTÜCK MIT AMORPHEN EIGENSCHAFTEN**

(71) Anmelder: Heraeus Amloy Technologies GmbH, 63450 Hanau (DE)
(72) Erfinder: WACHTER, Hans-Jürgen, 63450 Hanau (DE); MILKE, Eugen, 63450 Hanau (DE); SHAKUR SHAHABI, Hamed, 63450 Hanau (DE)
(74) Vertreter: Kilchert, Jochen

(57) **Zusammenfassung**

Simulationssystem (2) zur Auswahl einer Legierung (L) sowie eines Fertigungsverfahrens (V) für ein zu fertigendes Werkstück (4) mit amorphen Eigenschaften, wobei das System (2) aufweist:
- eine Eingabeeinheit (6), zur Eingabe eines Anforderungsprofils (A) für das zu fertigende Werkstück (4),
- zumindest eine Speichereinheit (8), die dazu ausgebildet ist, Informationsdaten (I) zu speichern, wobei die Informationsdaten (I) Informationen über physikalische und/oder chemische und/oder mechanische Eigenschaften mehrerer Legierungen (L) zur Herstellung von Werkstücken (4) mit amorphen Eigenschaften und Informationen über Fertigungsverfahren (V) angeben,
- eine Analyseeinheit (10), die derart eingerichtet ist,
∘ in Abhängigkeit des Anforderungsprofils (A) sowie der Informationsdaten (I) zur Erstellung von Simulationsdaten mehrere Werkstücke (4) zu simulieren,
∘ auf Basis der Simulationsdaten sowie des Anforderungsprofils (A) die simulierten Werkstücke zu bewerten;
∘ auf Basis der Bewertung eine Legierung (L) sowie ein Fertigungsverfahren (V) für das zu fertigende Werkstück (4) auszuwählen,
eine Ausgabeeinheit (20), die dazu ausgebildet ist, die ausgewählte Legierung (L) sowie das ausgewählte Fertigungsverfahren (V) auszugeben

## Beschreibung

Die Erfindung betrifft ein Simulationssystem sowie ein Verfahren zur Auswahl einer Legierung sowie eines Fertigungsverfahrens für ein zu fertigendes Werkstück mit amorphen Eigenschaften, ein computerlesbares-Speichermedium, eine Fertigungsanlage zur Fertigung eines Werkstücks mit amorphen Eigenschaften sowie ein Steuerverfahren.

Amorphe Metalle sind eine neuartige Werkstoffklasse, die bei anderen Werkstoffen nicht realisierbare physikalische Eigenschaften bzw. Kombinationen von Eigenschaften aufweisen.

Von amorphen Metallen spricht man, wenn Metalllegierungen auf atomarer Ebene keine kristalline, sondern eine amorphe Struktur aufweisen. Die für Metalle ungewöhnliche amorphe Atomanordnung führt zu einzigartigen Kombinationen physikalischer Eigenschaften. Amorphe Metalle sind im Allgemeinen härter, korrosionsbeständiger und fester als gewöhnliche Metalle bei gleichzeitig hoher Elastizität. Durch Abwesenheit von Korngrenzen ergibt sich weniger chemische Angriffsfläche, sodass metallische Gläser weniger korrosionsanfällig sind.

Seit ihrer Entdeckung am California Institute of Technology sind metallische Gläser, wie amorphe Metalle auch genannt werden, Gegenstand umfangreicher Forschung. Im Laufe der Jahre gelang es, die Prozessierbarkeit und Eigenschaften dieser Materialklasse kontinuierlich zu verbessern. Waren die ersten metallischen Gläser noch einfache, binäre (aus zwei Komponenten aufgebaute) Legierungen, deren Herstellung Abkühlraten im Bereich von 106 Kelvin pro Sekunde (K/s) erforderten, lassen sich neuere, komplexere Legierungen bereits bei deutlich geringeren Abkühlraten im Bereich einiger K/s in den Glaszustand überführen. Dies hat erheblichen Einfluss auf die Prozessführung sowie die realisierbaren Werkstücke. Die Abkühlgeschwindigkeit, ab der eine Kristallisation der Schmelze ausbleibt und die Schmelze im Glaszustand erstarrt, wird als kritische Abkühlrate bezeichnet. Die kritische Abkühlrate ist eine systemspezifische, stark von der Zusammensetzung der Schmelze abhängige Größe, welche zudem die maximal erreichbaren Bauteildicken festlegt. Bedenkt man, dass die in der Schmelze gespeicherte Wärmeenergie ausreichend schnell durch das System abtransportiert werden muss, wird klar, dass sich aus Systemen mit hohen kritischen Abkühlraten lediglich Werkstücke mit geringer Dicke fertigen lassen. Anfänglich wurden metallische Gläser daher meist nach dem Schmelzspinnverfahren (Englisch: "melt spinning") hergestellt. Die Schmelze wird hierbei auf ein rotierendes Kupferrad abgestreift und erstarrt glasartig in Form von dünnen Bändern bzw. Folien mit Dicken im Bereich einiger hundertstel bis zehntel Millimeter. Durch die Entwicklung neuer, komplexer Legierungen mit deutlich geringeren kritischen Abkühlraten, können zunehmend andere Herstellungsverfahren genutzt werden. Heutige massivglasbildende metallische Legierungen lassen sich bereits durch Gießen einer Schmelze in gekühlte Kupferkokillen in den Glaszustand überführen. Die realisierbaren Bauteildicken liegen dabei legierungsspezifisch im Bereich einiger Millimeter bis Zentimeter. Derartige Legierungen werden als metallische Massivgläser (Englisch: "bulk metallic glasses", BMG) bezeichnet. Heutzutage ist eine Vielzahl solcher Legierungssysteme bekannt.

Die Unterteilung metallischer Massivgläser erfolgt gewöhnlich anhand der Zusammensetzung, wobei man das Legierungselement mit dem höchsten Gewichtsanteil als Basiselement bezeichnet. Die bestehenden Systeme umfassen beispielsweise Edelmetall-basierte Legierungen wie Gold-, Platin, und Palladium-basierte metallische Massivgläser, frühe Übergangsmetall-basierte Legierungen wie z.B. Titan- oder Zirkoniumbasierte metallische Massivgläser, späte Übergangsmetall-basierte Systeme auf Basis von Kupfer-, Nickel- oder Eisen, aber auch Systeme auf Basis von seltenen Erden, z.B. Neodym oder Terbium.

Metallische Massivgläser weisen im Vergleich zu klassischen kristallinen Metallen typischer Weise folgende Eigenschaften auf:
- eine höhere spezifische Festigkeit, was zum Beispiel dünnere Wandstärken ermöglicht,
- eine höhere Härte, wodurch die Oberflächen besonders kratzfest sein können,
- eine viel höhere elastische Dehnbarkeiten und Resilienzen,
- eine thermoplastische Formbarkeit und
- eine höhere Korrosionsbeständigkeit.

Aufgrund ihrer vorteilhaften Eigenschaften wie z.B. einer hohen Festigkeit und dem Ausbleiben einer Erstarrungsschwindung sind metallische Gläser, insbesondere metallische Massivgläser, sehr interessante Konstruktionswerkstoffe, die sich prinzipiell für die Herstellung von Bauteilen in Serienfertigungsverfahren wie dem Spritzguss eignen, ohne dass weitere Bearbeitungsschritte nach erfolgter Formgebung zwingend erforderlich wären. Um beim Abkühlen aus der Schmelze eine Kristallisation der Legierung zu verhindern, muss eine kritische Abkühlgeschwindigkeit überschritten werden. Je größer jedoch das Volumen der Schmelze ist, desto langsamer (bei ansonsten unveränderten Bedingungen) kühlt die Schmelze ab. Wird eine bestimmte Probendicke überschritten, kommt es zu einer Kristallisation, bevor die Legierung amorph erstarren kann.

Neben den hervorragenden mechanischen Eigenschaften metallischer Gläser ergeben sich aus dem Glaszustand auch einzigartige Prozessierungsmöglichkeiten. So lassen sich metallische Gläser nicht nur durch schmelzmetallurgische Verfahren formen, sondern auch über ein thermoplastisches Formen bei vergleichsweise niedrigen Temperaturen analog zu thermoplastischen Kunststoffen oder Silikatgläsern formgebend verarbeiten. Hierzu wird das metallische Glas zunächst über den Glasübergangspunkt erwärmt und verhält sich dann wie eine hochviskose Flüssigkeit, die bei relativ niedrigen Kräften umgeformt werden kann. Im Anschluss an die Verformung wird das Material wieder unter die Glasübergangtemperatur abgekühlt.

Bei der Verarbeitung von amorphen Metallen wird die natürliche Kristallisation durch rasches Abkühlen (Einfrieren im schmelzflüssigen Zustand) der Schmelze unterbunden, so dass den Atomen die Beweglichkeit genommen wird, bevor sie eine Kristallanordnung einnehmen können. Viele Eigenschaften kristalliner Materialen werden durch Störungen im atomaren Aufbau, sogenannte Gitterdefekte (Leerstellen, Versetzungen, Korngrenzen, Phasengrenzen, etc.) beeinflusst bzw. bestimmt.

Durch das schnelle Abkühlen wird der Schrumpf des Materials reduziert, sodass bei amorphen Metallen genauere Bauteilgeometrien erreicht werden können. Plastische Verformung erfolgt erst bei Dehnungen über 1.8 %. Im Vergleich dazu verformen sich kristalline, metallische Werkstoffe in der Regel bereits bei deutlichen geringeren Dehnungen (<0.5 %) irreversibel. Die Kombination aus hoher Streckgrenze bei hoher elastischer Dehnung resultiert zudem in einem hohen Speichervermögen an elastischer Energie.

Die Wärmeleitfähigkeit des verwendeten Materials setzt der Abkühlgeschwindigkeit jedoch eine physikalische Grenze, da die im Bauteil enthaltene Wärme über die Oberfläche an die Umgebung abgegeben werden muss. Dies führt zu Einschränkungen in der Herstellbarkeit von Bauteilen und in der Anwendbarkeit von Herstellungsverfahren.

Es sind unterschiedliche Verfahren zur Herstellung von Werkstücken aus amorphen Metallen bekannt. So ist es möglich, Werkstücke unter Verwendung von additiven Fertigungsverfahren, wie dem 3D-Druck herzustellen. Dabei können die amorphen Eigenschaften des Werkstücks durch das Einstellen der Prozessparameter, wie der Scan-Geschwindigkeit, der Energie des Laserstrahls oder des abzufahrenden Musters, sichergestellt werden.

Ein Vorteil der additiven Fertigungstechnik ist, dass prinzipiell jede erdenkliche Geometrie realisiert werden kann. Ferner kann es von Vorteil sein, dass bei additiven Fertigungsverfahren kein gesonderter Kühlprozess notwendig ist, da durch das schichtweise Herstellen des Werkstücks und einer Einstellung der Größe des Schmelzpools über Laserenergie und Verfahrweg des Lasers eine gute Auskühlung gewährleistet sein kann.

Nachteilig bei additiven Fertigungsverfahren sind die zum Zeitpunkt der Anmeldung geringe Aufbauraten gerade bei großdimensionierten Werkstücken. Ferner muss für einige Anwendungen, also für bestimmte zu fertigende Werkstücke, hochreines Pulvermaterial als Ausgangsmaterial für den additiven Fertigungsprozess verwendet werden. Sind Verunreinigungen in dem Material vorhanden, so kann es an den Stellen der Verunreinigung zu Kristallisation kommen, d.h. zu nicht amorphen Metall, welches zu einer Verschlechterung der mechanischen und chemischen Eigenschaften führen kann. Oberflächennah kann es wegen der Verunreinigung notwendig sein, das Werkstück nachzubearbeiten, was aufwendig ist. Darüber hinaus kommt es bei der additiven Fertigung immer zu einer gewissen Rauigkeit an der Oberfläche des Werkstücks, sodass dies in den meisten Fällen durch Schleifen oder Fräsen nachbearbeitet werden muss.

Eine weitere Fertigungsmöglichkeit bietet das Spritzgießen. Dabei können zum Zeitpunkt der Anmeldung Gewichte von Werkstücken im Bereich von 80-120g oder größer realisiert werden. Üblicherweise wird das zu verwendende Material mittels induktivem Aufheizen innerhalb von ca. 10-60 Sekunden auf ca. 900-1100°C erhitzt und homogenisiert.

Nach dem Aufheizen wird das schmelzflüssige Material mittels eines Stempels in eine Form gedrückt. Dabei ist es für die Materialeigenschaften wichtig, dass wenn die Form vollständig mit Material gefüllt ist, das Material innerhalb der Form überall eine Temperatur oberhalb des Materialschmelzpunktes aufweisen sollte. Um amorphe Materialeigenschaften zu erzielen muss das flüssige Material innerhalb der Form anschließend schnell auf unterhalb der Glasumwandlungstemperatur abgekühlt werden.

Die möglichen Geometrien beim Spritzgießen sind aufgrund der Abkühlgeschwindigkeit des Materials auf Wandstärken von 0,3 -7,0 mm begrenzt. Bei größeren Wandstärken ist die Abkühlgeschwindigkeit zu gering, sodass sich kristalline Strukturen ausbilden bevor das Material auf unterhalb der Glasumwandlungstemperatur abgekühlt ist. Bei kleineren Wandstärken kühlt das Material abhängig von der zu füllenden Länge zu schnell ab und erstarrt bevor die Form vollständig gefüllt ist.

Um schon im Vorfeld bei der Konstruktion, der Dimensionierung, der Auswahl des Legierungswerkstoffes, der Auswahl des Herstellungsverfahrens oder Ähnlichem sicherzugehen, dass die dem Werkstoff zugeführte Wärmemenge an die Umgebung hinreichend schnell abgegeben werden kann, kann das Abkühlverhalten simuliert und analysiert werden.

Die DE 10 2015 110 591 A1 beschreibt beispielsweise eine Einrichtung und ein Herstellungserzeugnis zum Vorhersagen von Materialeigenschaften einer gegossenen aluminiumbasierten Komponente. Hierbei umfasst ein computerbasiertes System zahlreiche Berechnungsmodule, die programmtechnisch derart miteinander zusammenwirken, dass die Module beim Empfangen von Daten, die der gegossenen aluminiumbasierten Komponente entsprechen, Leistungsmerkmale des Materials bereitstellen.

Hierbei nachteilig ist, dass die beschriebene Einrichtung lediglich zur Auswahl einer Legierung und nicht zusätzlich zur Auswahl eines Fertigungsverfahrens ausgebildet ist. Wie bereits vorstehend ausführlich dargelegt, ist eine Auswahl einer geeigneten Legierung sowie eines geeigneten Fertigungsverfahrens für einen Anwender ohne dezidiertes Fachwissen über amorphe Metalle schwierig.

Die EP3246831 beschreibt, basierend auf numerischen Methoden, eine skalierbare und vorhersagende 3D-Drucksimulation für die Produktion von komplexen Bauteilen, wobei primär der Druckverlauf, die Druckzeit und die Kühlleistung auf Basis von lokalisierten Erwärmungseffekten simuliert werden. Eine Herstellbarkeitsanalyse des Bauteils, insbesondere in Bezug auf seinen schmelzflüssigen Zustand und das darauffolgende Abkühlen, wird nicht durchgeführt.

Es ist ferner aus der DE102006047806 bekannt, eine Abbildung einer Warmumformung einer Metallplatine aus einem umwandelbaren Stahlwerkstoff mit Hilfe der Finite-Elemente-Methode zu simulieren. Bei der Warmumformungssimulation werden nicht nur die mechanischen und physikalischen Eigenschaften des umzuformenden Stahlwerkstoffs berücksichtigt, sondern es werden im Rahmen einer komplexen thermischmechanisch gekoppelten Simulation Werkstoffdaten berücksichtigt, die in Form eines Zeit-Temperatur-Umwandlungs-Datensatzes des spezifischen Stahlwerkstoffs in das Verfahren einfließen. Auf diese Weise können die ermittelten temporären lokalen mechanischen Eigenschaftswerte auf Basis der jeweiligen Phasenzusammensetzung an ein Versagensmodell zur Verbesserung der Bauteilprognose und zur Prozessoptimierung übergeben werden.

Die DE102006047806 beschreibt somit ein Simulationsverfahren zur Abbildung einer Warmumformung einer Metallplatine aus einem umwandelbaren Stahlwerkstoff mit Hilfe der Finite-Elemente-Methode. Dabei stehen temporäre lokale mechanische Eigenschaften, z.B. die Härte und die physikalischen Eigenschaften der Metallplatte während und nach dem Ende der Warmformungssimulation auf der Grundlage der lokalen und temporären Phasenzusammensetzung des Stahlmaterials im Vordergrund.

Ein weiteres Beispiel ist aus der WO2018182513 bekannt, die ein computerimplementiertes Verfahren zur Beurteilung geometrischer Änderungen eines durch einen additiven Fertigungsprozess zu erstellenden Objekts beschreibt, wobei während dieses additiven Herstellungsprozesses ein kristallisierbares Material aus einem Pulver in eine Massenform umgewandelt wird und währenddessen der Gegenstand aus der Massenform gebildet wird.

Das Verfahren der WO2018182513 umfasst:
i. Bereitstellen einer Simulationsdomäne, die ein Finite-Elemente-Modell des Objekts umfasst, das in einen simulierten Kuchen des Pulvers eingebettet ist; wobei das Finite-Elemente-Modell finite Elemente des Objekts und finite Elemente des simulierten Kuchens des Pulvers umfasst;
ii. Zuordnen von thermischen Eigenschaften des volumenkristallharzierbaren Materials zu jedem finiten Element des Objekts;
iii. Zuordnen der thermischen Eigenschaften des pulverkristallisierbaren Materials zu jedem finiten Element des simulierten Kuchens des Pulvers;
iv. Zuordnen einer simulierten ersten Temperatur zu jedem finiten Element;
v. Durchführen einer Finite-Elemente-Analyse des Finite-Elemente-Modells unter einer simulierten Abkühlungsbedingung, wobei die simulierte Abkühlungsbedingung das Anlegen einer simulierten zweiten Temperatur an mindestens eine Grenze des Simulationsbereichs umfasst, wobei die simulierte zweite Temperatur niedriger als die simulierte erste Temperatur ist;

Dabei umfasst die Finite-Elemente-Analyse der WO2018182513 Folgendes:
i. Bestimmen eines simulierten kristallinen Volumenanteils des simulierten kristallisierbaren Volumenmaterials für jedes finite Element des Objekts;
ii. Bestimmen eines simulierten Wärmeausdehnungskoeffizienten für jedes finite Element des Objekts als Funktion des simulierten kristallinen Volumenanteils, des Wärmeausdehnungskoeffizienten (<3 ∼ 4) einer kristallinen Phase des kristallisierbaren Materials und des Wärmeausdehnungskoeffizienten einer amorphen Phase des kristallisierbaren Materials;
iii. Durchführen der Finite-Elemente-Analyse, bis ein Gleichgewichtszustand erreicht ist.

Die WO2018182513 beschreibt ein computerimplementiertes Verfahren zur Beurteilung geometrischer Änderungen eines durch einen additiven Fertigungsprozess herzustellenden Bauteils. Hierbei steht eine kristallisationsbedingte Volumenänderung im Vordergrund.

Aus den beschriebenen Nachteilen des Stands der Technik ergibt sich die Aufgabe, ein Simulationssystem zur einfachen Auswahl einer Legierung sowie eines Fertigungsverfahrens für ein zu fertigendes Werkstück mit amorphen Eigenschaften für einen Anwender bereitzustellen.

Die Aufgabe wird erfindungsgemäß gelöst durch ein Simulationssystem mit den Merkmalen des Anspruchs 1. Vorteilhafte Ausgestaltungen, Weiterbildungen und Varianten sind Gegenstand der Unteransprüche.

Insbesondere wird die Aufgabe gelöst durch ein Simulationssystem zur Auswahl einer Legierung sowie eines Fertigungsverfahrens für ein zu fertigendes Werkstück mit amorphen Eigenschaften, wobei das System eine Eingabeeinheit zur Eingabe eines Anforderungsprofils für das zu fertigende Werkstück aufweist.

Weiterhin weist das System zumindest eine Speichereinheit auf, die dazu ausgebildet ist, Informationsdaten zu speichern, wobei die Informationsdaten Informationen über physikalische und/oder chemische und/oder mechanische Eigenschaften mehrere Legierungen zur Herstellung von Werkstücken mit amorphen Eigenschaften angeben. Im Rahmen der nachfolgenden Ausführungen wird vereinfachend nicht einschränkend lediglich eine Speichereinheit erwähnt. Es können jedoch auch mehrere Speichereinheiten oder eine Speichereinheit mit mehreren Partitionen oder Modulen vorgesehen sein.

Weiterhin geben die Informationsdaten auch Informationen über Fertigungsverfahren an, insbesondere zur Fertigung derartiger Werkstücke.

Zudem weist das Simulationssystem eine Analyseeinheit auf. Die Analyseeinheit ist derart eingerichtet, in Abhängigkeit des Anforderungsprofils sowie der Informationsdaten zur Erstellung von Simulationsdaten mehrere Werkstücke zu simulieren. Weiterhin ist die Analyseeinheit dazu eingerichtet, auf Basis der Simulationsdaten sowie des Anforderungsprofils die simulierten Werkstücke zu bewerten.

Die Analyseeinheit ist ferner derart eingerichtet, auf Basis der Bewertung, eine Legierung sowie ein Fertigungsverfahren für das zu fertigende Werkstück auszuwählen. Diese Auswahl erfolgt vorzugsweise derart, dass eine Legierung und ein Fertigungsverfahren ausgewählt werden, deren simuliertes Werkstück - also ein Werkstück, welches aus dieser Legierung mit einem in der Speichereinheit hinterlegten Fertigungsverfahren simuliert wurde - dem des herzustellenden Werkstückes bzw. dessen Anforderungsprofil im Wesentlichen entspricht oder im Idealfall vollständig entspricht. Im Rahmen dieser Anmeldung kann im Wesentlichen eine Identität mit einer Abweichung von kleiner gleich 1%, 5%, 10%, 20%, 25% oder 30% von einer Masse, einem Volumen oder allgemein eines Parameters des zu fertigenden Werkstücks bedeuten.

Das Simulationssystem weist ebenfalls eine Ausgabeeinheit auf, die dazu ausgebildet ist, die ausgewählte Legierung sowie das ausgewählte Fertigungsverfahren für einen Anwender auszugeben. D.h. die Ausgabeeinheit kommuniziert die ausgewählte Legierung und/oder das ausgewählte Fertigungsverfahren an den Anwender.

Durch das erfindungsgemäße Simulationssystem ist somit eine Auswahl einer Legierung sowie eines Fertigungsverfahrens für ein zu fertigendes Werkstück mit amorphen Eigenschaften bereitgestellt, durch das ohne ein Expertenwissen des Anwenders eine geeignete Legierung sowie ein geeignetes Fertigungsverfahren für das Werkstück angegeben werden kann. Somit kann dem Anforderungsprofil bestmöglich entsprochen werden.

In einer Ausführungsform kann die Analyseeinheit eine Berechnungseinheit aufweisen, die dazu ausgebildet sein kann, durch das Anforderungsprofil angegebene Eigenschaften des zu fertigenden Werkstücks zu berechnen. Hierbei kann es sich insbesondere um Eigenschaften handeln, die nicht unmittelbar aus dem Anforderungsprofil des zu fertigenden Werkstücks hervorgehen, welche jedoch für die Auswahl einer geeigneten Legierung sowie eines geeigneten Fertigungsverfahrens wichtig sind. Hierdurch wird aufgrund einer größeren Informationsdichte die Simulation sowie die anschließende Auswahl einer geeigneten Legierung sowie eines geeigneten Fertigungsverfahrens präzisiert.

In einer Ausführungsform kann die Analyseeinheit eine erste Simulationseinheit aufweisen, die dazu ausgebildet sein kann, in Abhängigkeit des Anforderungsprofils sowie der berechneten Eigenschaften legierungsabhängig eine mechanische Beanspruchung des zu fertigenden Werkstücks zu simulieren und Informationen über die simulierte mechanische Beanspruchung zu den Simulationsdaten hinzuzufügen. Unter der mechanischen Beanspruchung kann beispielsweise eine (geforderte) biege- und/oder Torsionsresistenz bzw. -beanspruchung des zu fertigenden Werkstücks verstanden werden. Alternativ kann unter der mechanischen Beanspruchung auch mechanische Eigenschaften wie beispielsweise eine Steifigkeit des zu fertigenden Werkstücks verstanden werden.

In einer weiteren Ausführungsform kann die Analyseeinheit eine zweite Simulationseinheit aufweisen, die dazu ausgebildet sein kann, in Abhängigkeit des Anforderungsprofils und der Informationsdaten chemische Eigenschaften des zu fertigenden Werkstücks legierungsabhängig zu simulieren und Informationen über die simulierten chemischen Eigenschaften zu den Simulationsdaten hinzuzufügen. Unter den chemischen Eigenschaften kann hierbei beispielsweise ein Korrosionsverhalten oder eine Medienbeständigkeit, insbesondere, wenn das zu fertigende Werkstück Säuren und/oder Basen ausgesetzt sein wird.

Alternativ oder ergänzend kann die Analyseeinheit in einer weiteren Ausführungsform eine dritte Simulationseinheit aufweisen. Die dritte Simulationseinheit kann dazu ausgebildet sein, in Abhängigkeit des Anforderungsprofils eine Fertigung des Werkstücks mittels der in der zumindest einen Speichereinheit enthaltenen Fertigungsverfahren zu simulieren. Hierbei werden somit wie bereits vorstehend erwähnt, Werkstücke simuliert, welche mit den auf der zumindest eine Speichereinheit enthaltenen Fertigungsverfahren simuliert hergestellt werden. Die Informationen über die simulierten Fertigungen können ebenfalls, wie in den bereits vorstehenden zwei Ausführungsformen, zu den Simulationsdaten hinzugefügt werden.

Der Vorteil hierbei ist in einer Präzisierung sowie Erhöhung der Informationsdaten und somit in einer Erhöhung der Quantität in Bezug auf die Informationsdaten zu sehen, wodurch das Simulationssystem mehr Informationen für die Auswahl der geeigneten Legierung sowie des geeigneten Fertigungsverfahrens zur Verfügung hat und somit eine genauere Auswahl erfolgen kann.

Die erste, zweite und dritte Simulationseinheit kann als eine einzige Einheit ausgebildet sein, bzw. kann es sich um eine logische Einteilung handeln. Diese können aber als eine einzige Datenstruktur bzw. als eine Funktion oder mehrere Funktionen eines Programms ausgebildet sein.

Bevorzugt kann das Anforderungsprofil geometrische und/oder mechanische und/oder chemische Eigenschaften des zu fertigenden Werkstücks angeben. So kann beispielsweise unter den geometrischen Eigenschaften eine Abmessung und/oder ein Gewicht des zu fertigenden Werkstückes verstanden werden. Speziell das Gewicht des zu fertigenden Werkstückes hat beispielsweise einen Einfluss auf ein geeignetes Fertigungsverfahren. Während zum Beispiel Werkstücke mit einem Gewicht zwischen 80gr und 100gr bevorzugt mittels eines Spritzgussverfahrens hergestellt werden können, werden Werkstücke mit einer von dieser vorstehend genannten Gewichtsspanne abweichenden Masse zweckdienlicherweise mittels eines 3-D-Druckverfahrens hergestellt.

Auch bieten sich manche Fertigungsverfahren und/oder manche Legierungen für komplexe geometrische Strukturen des zu fertigenden Werkstückes bevorzugt an, während andere Legierungen und/oder Fertigungsverfahren für etwaige geometrische Strukturen unzweckmäßig sind. Unter den mechanischen Eigenschaften können vorzugsweise jedoch nicht abschließend die bereits vorstehend beschriebenen mechanischen Eigenschaften des zu fertigenden Werkstückes verstanden werden.

Ebenfalls können unter den chemischen Eigenschaften des zu fertigenden Werkstückes die bereits vorstehend genannten chemischen Eigenschaften verstanden werden. Hierunter kann jedoch auch beispielsweise eine Qualität der auszuwählenden Legierung verstanden werden, also z.B. speziell ein Sauerstoffgehalt der jeweiligen Legierung. Diese chemische Eigenschaft ist zweckdienlicherweise in Bezug auf eine Größe des zu fertigenden Werkstücks zu betrachten, da vorzugsweise bei großen zu fertigenden Werkstücken(z.B. 2-6mm bei Zr basierten Legierungen) auch höhere Anforderungen an die Qualität des Ausgangselements gesetzt werden. Bei kleineren zu fertigenden Werkstücken (z.B. <2mm bei Zr basierten Legierungen) ist im Hinblick auf eine Kosteneffizienz die Qualität und speziell der Sauerstoffgehalt der Legierung unter Umständen vernachlässigbar. Weiterhin kann jedoch unter der chemischen Eigenschaft auch eine Biokompatibilität der Legierung verstanden werden, welche sich insbesondere im medizinischen Bereich bzw. bei medizinischen Anwendungen niederschlägt. So haben sich speziell kupferfreie Legierungen im medizinisch-technischen Bereich als vorteilhaft erwiesen. Bezüglich des bereits vorstehend erwähnten Korrosionsverhaltens aber auch im Hinblick auf eine sogenannte metallische Ionenfreigabe, beispielsweise bei einer Schweißentwicklung, haben sich speziell zirkonium-, titan- oder platinbasierte Legierungen als vorteilhaft erwiesen.

Somit können die Informationsdaten in einer Ausführungsform Informationen über zumindest eine physikalische und/oder chemische und/oder mechanische Eigenschaften angeben. Insbesondere handelt es sich hierbei um die nachfolgend aufgeführten Eigenschaftsgruppen:
- Thermische Eigenschaften der Legierung,
- Medienbeständigkeit der Legierung,
- Chemische Eigenschaften,
- Amorphizität in Abhängigkeit eines Verunreinigungsgrades (Sauerstoffgehalt),
- Beanspruchungsabhängige Alterungserscheinungen und/oder
- Abkühlverhalten der Legierung.

Speziell letztgenannte Eigenschaft, also das Abkühlverhalten der Legierung, bezieht sich vorzugsweise auf das auszuwählende Fertigungsverfahren, während die anderen vorstehend aufgezählten Eigenschaften/Eigenschaftsgruppen primär auf das zu fertigende Werkstück abzielen. Die Speichereinheit kann z.B. als Datenbank ausgebildet sein. Dann können die vorgenannten Eigenschaften beispielsweise als Datensatz in der Datenbank hinterlegbar oder hinterlegt sein.

In einer Ausführungsform können die Informationsdaten ergänzend Informationen über die Fertigungsschritte der Fertigungsverfahren enthalten. Hierbei kann es sich bei den Informationen, insbesondere um eine Stempelgeschwindigkeit und/oder eine Ausgangstemperatur der zu verarbeitenden Legierung handeln. Jedoch können auch andere prozesstechnische Informationen über die Fertigungsschritte des Fertigungsverfahrens Teil der Informationen sein. Somit wird die Auswahl insbesondere eines geeigneten Fertigungsverfahrens weiter verbessert.

In einer Ausführungsform kann die Analyseeinheit dazu ausgebildet sein, in Abhängigkeit des Anforderungsprofils eine Vorauswahl innerhalb der Speichereinheit gespeicherten Legierung und oder an innerhalb der Speichereinheit gespeicherten Fertigungsverfahren zu treffen. Zudem kann eine Auswahl einer geeigneten Legierung und/oder eines geeigneten Fertigungsverfahrens anhand dieser Vorauswahl erfolgen. Bei der Vorauswahl können beispielsweise chemische Anforderungen wie die bereits genannte Biokompatibilität der Legierung, aber auch mechanische Anforderungen, beispielsweise eine Abriebfestigkeit, eine Härte sowie elektrische Anforderungen und/oder auch magnetische Anforderungen berücksichtigt werden.

Unter der Vorauswahl kann somit verstanden werden, dass aufgrund dieser vorgenannten Eigenschaften bestimmte Legierungen und/oder Fertigungsverfahren bereits ohne eine Simulation ausscheiden, weil ungeeignete Legierungen oder Fertigungsverfahren nicht mehr für eine spätere Auswahl in Betracht kommen, sodass ein Simulationsaufwand minimiert wird.

In einer Ausführungsform kann die Analyseeinheit dazu ausgebildet sein, durch eine Zuordnung der ausgewählten Legierung sowie des ausgewählten Fertigungsverfahrens zu dem Anforderungsprofil ein Datenpaar zu generieren. Mit anderen Worten kann einem Anforderungsprofil eine ausgewählte Legierung sowie ein ausgewähltes Fertigungsverfahren zugeordnet werden und diese Zuordnung kann in Form eines Datenpaares in der Speichereinheit hinterlegt werden. Weiterhin kann die Analyseeinheit in dieser Ausführungsform dazu ausgebildet sein, bei Eingabe eines in der Speichereinheit gespeicherten Anforderungsprofils durch den Nutzer eine dem Anforderungsprofil zugeordnete Legierung und ein zugeordnetes Fertigungsverfahren anzugeben. Somit kann im Falle der Eingabe eines derartigen Anforderungsprofils auf eine Simulation verzichtet werden und sofort eine geeignete Legierung sowie ein geeignetes Verfahren vorgeschlagen und ausgegeben werden.

Der Vorteil hierbei ist in dem deutlich reduzierten Simulationsaufwand zu sehen. Ebenfalls kann hierbei das Simulationssystem und eine Auswahl einer geeigneten Legierung und/oder eines geeigneten Fertigungsverfahrens zum einen beschleunigt und zum anderen vereinfacht werden.

Es wird weiterhin im Rahmen dieser Anmeldung ein Verfahren zur Auswahl einer Legierung sowie eines Fertigungsverfahrens für ein fertigendes Werkstück mit amorphen Eigenschaften offenbart und beansprucht, wobei das Verfahren folgende Schritte umfasst:
- Eingabe eines Anforderungsprofils des zu fertigenden Werkstückes, insbesondere mittels einer Eingabeeinheit,
- Berechnung von mechanischen und/oder chemischen und/oder physikalischen Parametern und Abgleich der berechneten Parameter mit gespeicherten Informationsdaten, die eine Information über physikalische und/oder chemische und/oder mechanische Eigenschaften mehrerer Legierungen zur Herstellung von Werkstücken mit amorphen Eigenschaften enthält,
- Simulation mehrerer Werkstücke in Abhängigkeit des Anforderungsprofils sowie der Informationsdaten,
- Erstellung von Simulationsdaten auf Basis der Simulationen,
- Bewertung der simulierten Werkstücke auf Basis der Simulationsdaten sowie des Anforderungsprofils,
- Auswählen einer Legierung sowie eines Fertigungsverfahrens für das zu fertigende Werkstück auf Basis der Bewertung,
- Ausgeben der ausgewählten Legierung sowie des ausgewählten Fertigungsverfahrens.

In einer Ausführungsform des Verfahrens können Eigenschaften des zu fertigenden Werkstücks, die durch das Anforderungsprofil angegeben werden, berechnet werden.

In einer Ausführungsform kann ein legierungsabhängiges Simulieren einer mechanischen Beanspruchung des zu fertigenden Werkstücks erfolgen. Dies kann in Abhängigkeit des Anforderungsprofils sowie der berechneten Eigenschaften erfolgen. Weiterhin können Informationen über die simulierte mechanische Beanspruchung zu den Simulationsdaten hinzugefügt werden.

In einer Ausführungsform können zudem chemische Eigenschaften des zu fertigenden Werkstücks in Abhängigkeit des Anforderungsprofils und der Information legierungsabhängiges simuliert und Informationen über diese simulierten chemischen Eigenschaften zu den Simulationsdaten hinzugefügt werden.

Alternativ oder ergänzend zu den vorgenannten Ausführungsformen kann eine Fertigung des Werkstücks in Abhängigkeit des Anforderungsprofils mittels der gespeicherten Fertigungsverfahren simuliert werden. Anschließend können Informationen über die simulierten Fertigungen zu den Simulationsdaten hinzugefügt werden.

In einer Ausführungsform können mittels der Informationsdaten Informationen über zumindest eine physikalische und/oder chemische und/oder mechanische Eigenschaften angegeben werden, insbesondere ausgewählt aus:
- thermische Eigenschaften der Legierung,
- Medienbeständigkeit der Legierung,
- chemische Eigenschaften,
- Amorphizität in Abhängigkeit eines Verunreinigungsgrades,
- beanspruchungsabhängige Alterungserscheinungen und/oder
- Abkühlverhalten der Legierung.

Hierdurch ist sichergestellt, dass zumindest die Eigenschaften, die zur Auswahl einer geeigneten Legierung und/oder eines geeigneten Verfahrens wichtig sind, angegeben werden und somit dem Verfahren zur Auswahl zugrunde liegen.

In einer weiteren Ausführungsform kann eine Vorauswahl an gespeicherten Legierungen und/oder gespeicherten Fertigungsverfahren in Abhängigkeit des Anforderungsprofils getroffen werden. Ferner kann in dieser Ausführungsform eine Auswahl einer Legierung und/oder eines Fertigungsverfahrens anhand der Vorauswahl erfolgen.

In einer Ausführungsform kann ein Datenpaar durch eine Zuordnung der ausgewählten Legierung sowie des ausgewählten Fertigungsverfahrens zu dem Anforderungsprofil generiert werden. Anschließend kann das generierte Datenpaar in der Speichereinheit gespeichert werden.

Erfolgt nun eine Eingabe eines Anforderungsprofils, welches bereits auf der Speichereinheit hinterlegt ist, wird vorzugsweise keine Simulation gestartet, sondern eine Ausgabe einer von dem gespeicherten Anforderungsprofil zugeordneten Legierung und eines dem Anforderungsprofil zugeordneten Fertigungsverfahrens.

Weiterhin wird die Aufgabe erfindungsgemäß gelöst durch ein computerlesbares Speichermedium, welches Instruktionen enthält, die mindestens einen Prozessor dazu veranlassen, ein Verfahren zu implementieren, wenn das Verfahren von den mindestens einen Prozessor ausgeführt wird. Bei dem Verfahren handelt es sich um das vorstehend beschriebene Verfahren zur Auswahl einer Legierung sowie eines Fertigungsverfahrens für ein zu fertigendes Werkstück mit amorphen Eigenschaften. Ferner wird eine Fertigungsanlage zur Fertigung eines Werkstücks mit amorphen Eigenschaften offenbart und beansprucht, wobei die Fertigungsanlage ein Simulationssystem zur Auswahl einer Legierung sowie eines Fertigungsverfahrens für das zu fertigende Werkstück aufweist. Bei dem Simulationssystem handelt es sich hierbei insbesondere um das bereits vorstehend im Rahmen dieser Anmeldung beschriebene Simulationssystem. Weiterhin weist die Fertigungsanlage eine Fertigungseinheit auf, die dazu ausgebildet ist, ein Werkstück unter Verwendung des Simulationssystems zu fertigen.

In einer Ausführungsform der Fertigungsanlage kann die Fertigungseinheit als eine Spritzgusseinrichtung oder als eine Einrichtung zur additiven Fertigung ausgebildet sein. Unter der Einrichtung zur additiven Fertigung kann hierbei beispielsweise eine 3-D Druck-Einrichtung verstanden werden. Hierdurch kann die Fertigungsanlage auf bzw. an verschiedene Anforderungen, insbesondere hinsichtlich einer Größe der zu fertigenden Werkstücke angepasst werden.

Zudem wird ein Steuerverfahren zur Steuerung einer Fertigungsanlage zur Fertigung eines Werkstücks mit amorphen Eigenschaften offenbart und beansprucht. Das Steuerverfahren dient insbesondere zur Steuerung der vorstehend beschriebenen Fertigungsanlage. Hierbei wird die Fertigungsanlage mit einer Legierung sowie einem Fertigungsverfahren betrieben, wobei sowohl die Legierung als auch das Fertigungsverfahren, insbesondere mit dem vorstehend beschriebenen Verfahren zur Auswahl einer Legierung sowie eines Fertigungsverfahrens ausgewählt sind.

Die im Hinblick auf das Simulationssystem aufgeführten Vorteile und bevorzugten Ausgestaltungen sind sinngemäß auf Verfahren zur Auswahl einer Legierung und/oder eines Fertigungsverfahrens, das computerlesbare Speichermedium sowie auf die Fertigungsanlage und deren Steuerverfahren zu übertragen und umgekehrt.

Das Simulationssystem und die Fertigungsanlage können auch räumlich getrennt voneinander angeordnet sein. Hierbei können das Simulationssystem und die Fertigungsanlage dann über ein Kommunikationsnetzwerk, z.B. dem Internet, miteinander kommunizieren. Die Ausgabe der geeigneten Legierung sowie des geeigneten Fertigungsverfahrens und/oder die Eingabe des Anforderungsprofils kann beispielsweise auf einer Website oder über eine Programmierschnittstelle, wie z.B. einer API erfolgen.

Im Folgenden wird die Erfindung anhand eines Ausführungsbeispiels näher erläutert. Dabei zeigen:
- Fig. 1: ein schematisiertes Simulationssystem zur Auswahl einer Legierung sowie eines Fertigungsverfahrens für ein zu fertigendes Werkstück mit amorphen Eigenschaften;
- Fig. 2: eine schematische Darstellung einer Fertigungseinheit sowie
- Fig. 3: eine schematische Darstellung eines Werkzeugs.

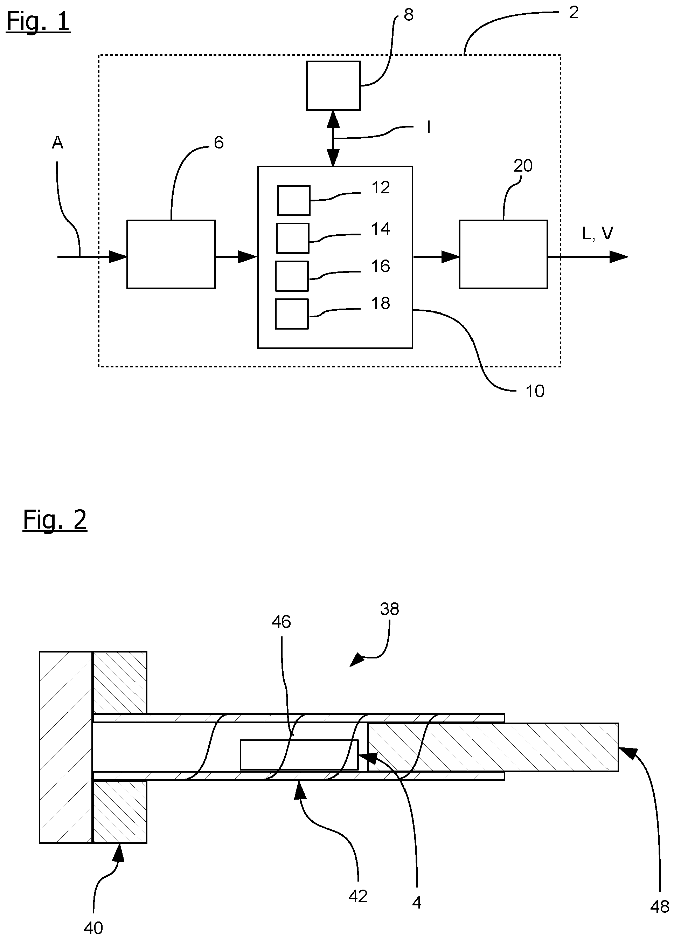

Fig. 1 zeigt ein schematisch dargestelltes Simulationssystem 2. Das Simulationssystem 2 ist zur Auswahl einer Legierung sowie eines Fertigungsverfahrens für ein zu fertigendes Werkstück 4 (vgl. Fig. 4) mit amorphen Eigenschaften ausgebildet.

Hierzu weist das Simulationssystem 2 gemäß Fig. 1 eine Eingabeeinheit 6 auf, die zur Eingabe eines Anforderungsprofils A für das zu fertigende Werkstück 4 dient. Hiermit ist es somit ermöglicht, dass ein Anwender die geforderten Eigenschaften des zu fertigenden Werkstückes 4 in Form des Anforderungsprofils A dem Simulationssystem 2 zur Verfügung stellt bzw. das Simulationssystem 2 mit dem Anforderungsprofil A speist.

Weiterhin weist das Simulationssystem 2 zumindest eine Speichereinheit 8 auf, die dazu ausgebildet ist, Informationsdaten I zu speichern, wobei die Informationsdaten I Informationen über physikalische und/oder chemische und/oder mechanische Eigenschaften mehrerer Legierungen L zur Herstellung von Werkstücken 4 sowie Informationen über Fertigungsverfahren V angeben. Die Speichereinheit 8 kann hierzu beispielsweise über mehrere Partitionen verfügen und/oder mehrteiligen ausgebildet sein, sich also aus mehreren Teilspeichereinheiten zusammensetzen. Weiterhin ist die Speichereinheit 8 kommunikativ und bidirektional mit einer Analyseeinheit 10 verbunden.

Die Analyseeinheit 10 ist hierbei derart eingerichtet, in Abhängigkeit des Anforderungsprofils A sowie der Informationsdaten I mehrere Werkstücke 4 zu simulieren. Dies dient zur Erstellung von Simulationsdaten. Weiterhin ist die Analyseeinheit 10 derart eingerichtet, auf Basis der Simulationsdaten sowie des Anforderungsprofils A die simulierten Werkstücke zu bewerten, bevor auf Basis der Bewertung einer Legierung L sowie ein Fertigungsverfahren V, welches im Hinblick auf das Anforderungsprofil A des zu fertigenden Werkstückes 4 besonders geeignet sind, ausgewählt werden.

Um die vorstehenden Verfahrensschritte zu realisieren, weist die Analyseeinheit 10 eine Berechnungseinheit 12 auf mittels der durch das Anforderungsprofil A angegebene Eigenschaften des zu fertigenden Werkstückes berechnet werden. Weiterhin weist die Analyseeinheit 10 eine erste Simulationseinheit 14 auf. Mittels der ersten Simulationseinheit 14 erfolgt eine Simulation einer mechanischen Beanspruchung des zu fertigenden Werkstückes 4 auf Basis und in Abhängigkeit des Anforderungsprofils A sowie der berechneten Eigenschaften. Hiernach werden die Informationen über die simulierte mechanische Beanspruchung zu den Simulationsdaten hinzugefügt.

In analoger Weise weist die Analyseeinheit 10 noch eine zweite Simulationseinheit 16 sowie eine dritte Simulationseinheit 18 auf. Mittels der zweiten Simulationseinheit 16 erfolgt eine Simulation chemischer Eigenschaften des zu fertigenden Werkstückes 4 in Abhängigkeit des Anforderungsprofils A sowie der Informationsdaten I. Mittels der dritten Simulationseinheit 18 wird eine Fertigung des Werkstücks 4 mittels der in der Speichereinheit 8 enthaltenen Fertigungsverfahren V simuliert. Sowohl die Informationen über die chemischen Eigenschaften, welche von der zweiten Simulationseinheit 16 bereitgestellt werden, als auch die Informationen über die simulierten Fertigungen, welche von der dritten Simulationseinheit 18 generiert werden, werden anschließend zu den Simulationsdaten hinzugefügt und für eine Auswahl einer geeigneten Legierung L sowie eines geeigneten Fertigungsverfahrens V im Rahmen der Bewertung durch die Analyseeinheit 10 herangezogen. Die Auswahl und die Bewertung erfolgt hierbei derart, dass die Legierung L und das Fertigungsverfahren V ausgewählt werden, die die durch das Anforderungsprofil A bestimmten Eigenschaften des zu fertigenden Werkstückes 4 erfüllen oder zumindest im Wesentlichen erfüllen. Falls mehrere Legierungen L und/oder mehrere Fertigungsverfahren V in Frage kommen, so wählt die Analyseeinheit 10 diejenige Legierung L und das Fertigungsverfahren V aus, welches im Hinblick auf übergeordnete Präferenzen, beispielsweise im Hinblick auf eine Kosteneffizienz, am geeignetsten sind.

Nach der Auswahl der geeigneten Legierung L sowie des geeigneten Fertigungsverfahrens V durch die Analyseeinheit 10 erfolgt eine Ausgabe der ausgewählten Legierung L sowie des ausgewählten Fertigungsverfahrens V durch eine Ausgabeeinheit 20. Die Ausgabeeinheit 20 kann hierbei beispielsweise eine optische Ausgabeeinheit 10 sein, bei der die Ausgabe auf einem Bildschirm erfolgt.

Gemäß einer Ausführungsform ist die Analyseeinheit 10 dazu ausgebildet, durch eine Zuordnung der ausgewählten Legierung L sowie des ausgewählten Fertigungsverfahrens V zu dem Anforderungsprofil ein Datenpaar zu generieren. Hierbei wird das generierte Datenpaar in der Speichereinheit 8 hinterlegt. Erfolgt nun eine Eingabe eines in der Speichereinheit 8 hinterlegten Anforderungsprofils A, so wird eine dem Anforderungsprofil A zugeordnete Legierung L und ein dem Anforderungsprofil A zugeordnetes Fertigungsverfahren V angegeben, ohne, dass eine Simulation stattfindet.

In einer Ausführungsform ist es denkbar, dass das Simulationssystem 2 ein künstliches neuronales Netz aufweist, um die Auswahl der geeigneten Legierung L und/oder des geeigneten Fertigungsverfahrens V zu optimieren.

Die Fig. 2 zeigt eine schematische Darstellung einer Fertigungseinheit 38, die als eine AMM (Amorphous-Metal) - Spritzgussanlage ausgebildet ist. Die Fertigungseinheit 38 umfasst eine Form im Werkzeug 40 und eine Schmelzkammer 42. Der Schmelzkammer 42 wird über einen Roboter ein massives Legierungssegment einer amorph erstarrenden Legierung (Rohling) 44 zugeführt und mittig in einer Induktionsspule 46 platziert. Der Rohling 44 (in Abbildung "4" statt "44") wird innerhalb der Schmelzkammer 42 mittels eines Heizelements insbesondere eines Induktionsfeldes, welches durch die Induktionsspule 46 erzeugt wird, erhitzt. Bei dem Rohling 44 handelt es sich um ein massives Legierungssegment einer amorph erstarrenden Legierung. Das Legierungssegment 44 weist zum Beispiel einen bestimmten Anteil Palladium, Platin, Zirconium, Titan, Kupfer, Aluminium, Magnesium, Niobium, Silizium und/oder Yttrium auf.

Durch das Heizelement bzw. die Induktionsspule 46 wird der Rohling 44 zum Schmelzen gebracht, sodass er in schmelzflüssiger Form vorliegt. Vorzugsweise wird der Rohling 44 auf eine Temperatur von 1050 °C aufgeheizt. Durch einen Kolben 48 wird das schmelzflüssige Material in das Werkzeug 40 eingespritzt.

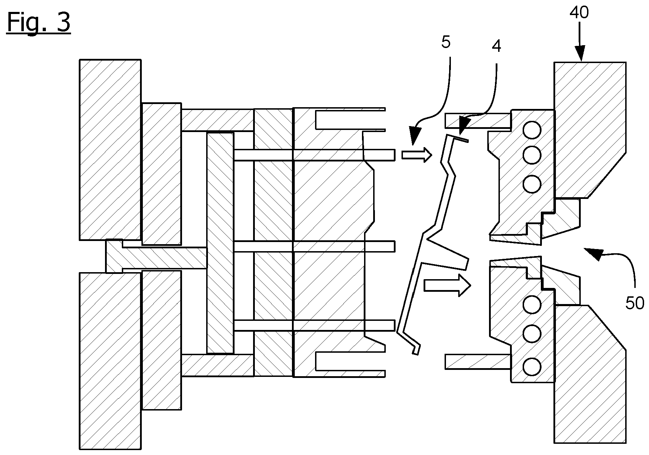

Fig. 3 zeigt den schematischen Aufbau eines Spritzwerkzeuges 40. Mittels einer oder einer Vielzahl von Öffnungen 50, die in eine Formkammer 52 des Werkzeuges 40 führen, wird die Formkammer 52 mit einer Schmelze gefüllt. Die Formkammer 52 ist als eine Negativform des herzustellenden Werkstücks 4 ausgelegt. In dem Ausführungsbeispiel der Figur 3 ist vorgesehen, dass eine Öffnung 50 dazu verwendet werden kann, flüssiges Material in die Formkammer 52 zu führen. Es kann vorteilhaft sein, mehrere Angüsse zur Füllung der Formkammer 52 zu nutzen, um eine gleichmäßige Temperaturverteilung zu erzielen als auch, um Verwirbelungen der Schmelze zu verringern. Eine gleichmäßige Temperaturverteilung und eine geringe Anzahl von Verwirbelungen führen zu einem besseren Kühlvorgang, zu einer homogenen Abkühlung und damit zu gleichmäßigen amorphen Materialeigenschaften.

Innerhalb der Formkammer 52 muss das flüssige Material schnell auskühlen, um eine Kristallisation zu verhindern. Das Auskühlen des flüssigen Materials hängt stark von der Geometrie des herzustellenden Bauteils bzw. Werkstücks 4 ab.

Die Erfindung ist nicht auf das vorstehend beschriebene Ausführungsbeispiel beschränkt. Vielmehr können auch andere Varianten der Erfindung von dem Fachmann hieraus abgeleitet werden, ohne den Gegenstand der Erfindung zu verlassen. Insbesondere sind ferner alle im Zusammenhang mit dem Ausführungsbeispiel beschriebenen Einzelmerkmale auch auf andere Weise miteinander kombinierbar, ohne den Gegenstand der Erfindung zu verlassen.

### Bezugszeichenliste:

- 2: Simulationssystem
- 4: Werkstück
- 6: Eingabeeinheit
- 8: Speichereinheit
- 10: Analyseeinheit
- 12: Berechnungseinheit
- 14: erste Simulationseinheit
- 16: zweite Simulationseinheit
- 18: dritte Simulationseinheit
- 20: Ausgabeeinheit
- 22: künstliches neuronales Netz
- 24: Eingangsdaten
- 26: Merkmalsdetektoren
- 28: erste Faltung
- 38: Fertigungseinheit
- 40: Werkzeug
- 42: Schmelzkammer
- 44: Rohling einer amorph erstarrenden Legierung
- 46: Induktionsspule
- 48: Kolben
- 50: Öffnung
- 52: Formkammer

- A: Anforderungsprofil
- I: Informationsdaten
- L: Legierung
- V: Fertigungsverfahren

## Patentansprüche

1. Simulationssystem (2) zur Auswahl einer Legierung (L) sowie eines Fertigungsverfahrens (V) für ein zu fertigendes Werkstück (4) mit amorphen Eigenschaften, wobei das System (2) aufweist:
- eine Eingabeeinheit (6), zur Eingabe eines Anforderungsprofils (A) für das zu fertigende Werkstück (4),
- zumindest eine Speichereinheit (8), die dazu ausgebildet ist, Informationsdaten (I) zu speichern, wobei die Informationsdaten (I) Informationen über physikalische und/oder chemische und/oder mechanische Eigenschaften mehrerer Legierungen (L) zur Herstellung von Werkstücken (4) mit amorphen Eigenschaften und Informationen über Fertigungsverfahren (V) angeben,
- eine Analyseeinheit (10), die derart eingerichtet ist,
∘ in Abhängigkeit des Anforderungsprofils (A) sowie der Informationsdaten (I) zur Erstellung von Simulationsdaten mehrere Werkstücke (4) zu simulieren,
∘ auf Basis der Simulationsdaten sowie des Anforderungsprofils (A) die simulierten Werkstücke zu bewerten,
∘ auf Basis der Bewertung eine Legierung (L) sowie ein Fertigungsverfahren (V) für das zu fertigende Werkstück (4) auszuwählen,
- eine Ausgabeeinheit (20), die dazu ausgebildet ist, die ausgewählte Legierung (L) sowie das ausgewählte Fertigungsverfahren (V) auszugeben.

2. Simulationssystem (2) nach Anspruch 1,
**dadurch gekennzeichnet, dass**
die Analyseeinheit (10) eine erste Simulationseinheit (14) aufweist, die dazu ausgebildet ist, in Abhängigkeit des Anforderungsprofils (A) sowie der berechneten Eigenschaften legierungsabhängig eine mechanische Beanspruchung des zu fertigenden Werkstücks (4) zu simulieren und Informationen über die simulierte mechanische Beanspruchung zu den Simulationsdaten hinzuzufügen.

3. Simulationssystem (2) nach einem der vorhergehenden Ansprüche,
**dadurch gekennzeichnet, dass**
die Analyseeinheit (10) eine zweite Simulationseinheit (16) aufweist, die dazu ausgebildet ist, in Abhängigkeit des Anforderungsprofils (A) und der Informationsdaten (I) chemische Eigenschaften des zu fertigenden Werkstücks (4) legierungsabhängig zu simulieren und Informationen über die simulierten chemischen Eigenschaften zu den Simulationsdaten hinzuzufügen.

4. Simulationssystem (2) nach einem der vorhergehenden Ansprüche,
**dadurch gekennzeichnet, dass**
die Informationsdaten (I) Informationen über zumindest eine physikalische und/oder chemische und/oder mechanische Eigenschaft angeben, insbesondere ausgewählt aus:
- thermische Eigenschaften der Legierung,
- Medienbeständigkeit der Legierung,
- chemische Eigenschaften,
- Amorphizität in Abhängigkeit eines Verunreinigungsgrades,
- beanspruchungsabhängige Alterungserscheinungen und/oder
- Abkühlverhalten der Legierung.

5. Simulationssystem (2) nach einem der vorhergehenden Ansprüche,
**dadurch gekennzeichnet, dass**
die Analyseeinheit (10) dazu ausgebildet ist, in Abhängigkeit des Anforderungsprofils (A) eine Vorauswahl an innerhalb der zumindest einen Speichereinheit (8) gespeicherten Legierungen (L) und/oder an innerhalb der zumindest einen Speichereinheit (8) gespeicherten Fertigungsverfahren (V) zu treffen und anhand der Vorauswahl eine Auswahl einer Legierung (L) und/oder eines Fertigungsverfahrens (V) erfolgt.

6. Simulationssystem (2) nach einem der vorhergehenden Ansprüche,
wobei die Analyseeinheit (10) dazu ausgebildet ist,
- durch eine Zuordnung der ausgewählten Legierung (L) sowie des ausgewählten Fertigungsverfahrens (V) zu dem Anforderungsprofil (A) ein Datenpaar zu generieren,
- das generierte Datenpaar in der zumindest einen Speichereinheit (8) zu hinterlegen,
- bei Eingabe eines in der zumindest einen Speichereinheit (8) gespeicherten Anforderungsprofils (A), eine dem Anforderungsprofil (A) zugeordnete Legierung (L) und ein dem Anforderungsprofil (A) zugeordnetes Fertigungsverfahren (V) anzugeben.

7. Verfahren zur Auswahl einer Legierung (L) sowie eines Fertigungsverfahrens (V) für ein zu fertigendes Werkstück (4) mit amorphen Eigenschaften, umfassend die Schritte:
- Eingabe eines Anforderungsprofils (A) des zu fertigenden Werkstückes (4), insbesondere mittels einer Eingabeeinheit (6),
- Berechnung von mechanischen und/oder chemischen und/oder physikalischen Parametern und Abgleich der berechneten Parameter mit gespeicherten Informationsdaten (I), die eine Information über physikalische und/oder chemische und/oder mechanische Eigenschaften mehrerer Legierungen (L) zur Herstellung von Werkstücken (4) mit amorphen Eigenschaften enthält,
- Simulation mehrerer Werkstücke (4) in Abhängigkeit des Anforderungsprofils (A) sowie der Informationsdaten (I),
- Erstellung von Simulationsdaten auf Basis der Simulationen sowie
- Bewertung der simulierten Werkstücke auf Basis der Simulationsdaten sowie des Anforderungsprofils (A),
- Auswählen einer Legierung (L) sowie eines Fertigungsverfahrens (V) für das zu fertigende Werkstück (4) auf Basis der Bewertung,
- Ausgeben der ausgewählten Legierung (L) sowie des ausgewählten Fertigungsverfahrens (V).

8. Verfahren nach Anspruch 7,
**gekennzeichnet durch**
Berechnen von Eigenschaften des zu fertigenden Werkstücks (4), die durch das Anforderungsprofil (A) angeben werden.

9. Verfahren nach einem der Ansprüche 7 bis 8,
**dadurch gekennzeichnet, dass**
die Informationsdaten (I) Informationen über zumindest eine physikalische und/oder chemische und/oder mechanische Eigenschaften angeben, insbesondere ausgewählt aus:
- thermische Eigenschaften der Legierung (L),
- Medienbeständigkeit der Legierung (L),
- chemische Eigenschaften,
- Amorphizität in Abhängigkeit eines Verunreinigungsgrades,
- beanspruchungsabhängige Alterungserscheinungen und/oder
- Abkühlverhalten der Legierung (L).

10. Verfahren nach einem der Ansprüche 7 bis 9,
**gekennzeichnet durch**
- Treffen einer Vorauswahl an gespeicherten Legierungen (L) und/oder gespeicherten Fertigungsverfahren (V) in Abhängigkeit des Anforderungsprofils (A),
- Auswählen einer Legierung (L) und/oder eines Fertigungsverfahrens (V) anhand der Vorauswahl.

11. Verfahren nach einem der Ansprüche 7 bis 10,
**dadurch gekennzeichnet, dass**
- Generieren eines Datenpaars durch eine Zuordnung der ausgewählten Legierung (L) sowie des ausgewählten Fertigungsverfahrens (V) zu dem Anforderungsprofil (A),
- Speichern des generierten Datenpaars,
- Ausgeben einer einem gespeicherten Anforderungsprofil (A) zugeordneten Legierung (L) und eines dem Anforderungsprofil (A) zugeordneten Fertigungsverfahrens (V), wenn das Anforderungsprofil (A) bereits gespeichert ist.

12. Computerlesbares-Speichermedium, welches Instruktionen enthält, die mindestens einen Prozessor dazu veranlassen, ein Verfahren nach einem der Ansprüche 7 bis 11 zu implementieren, wenn das Verfahren von dem mindestens einen Prozessor ausgeführt wird.

13. Fertigungsanlage zur Fertigung eines Werkstücks mit amorphen Eigenschaften, aufweisend:
- ein Simulationssystem (2) zur Auswahl einer Legierung (L) sowie eines Fertigungsverfahrens (V) für das zu fertigende Werkstück (4), insbesondere nach einem der Ansprüche 1 bis 6 sowie
- eine Fertigungseinheit (38) die dazu ausgebildet ist, ein Werkstück (4) unter Verwendung des Simulationssystems (2) zu fertigen.

14. Fertigungsanlage nach Anspruch 13,
**dadurch gekennzeichnet, dass**
die Fertigungseinheit (38) als eine Spritzgusseinrichtung oder als eine Einrichtung zur additiven Fertigung ausgebildet ist.

15. Steuerverfahren zur Steuerung einer Fertigungsanlage zur Fertigung eines Werkstücks (4) mit amorphen Eigenschaften, insbesondere zur Steuerung einer Fertigungsanlage nach einem der Ansprüche 13 oder 14,
**dadurch gekennzeichnet, dass**
die Fertigungsanlage mit einer Legierung (L) sowie einem Fertigungsverfahren (V) betrieben wird, welche insbesondere mit einem Verfahren nach einem der Ansprüche 7 bis 11 ausgewählt sind.
